# EUROPEAN PATENT APPLICATION

(11) **EP 4 316 364 A1**
(43) Date of publication of application: **07.02.2024**
(21) Application number: 22774919.9
(22) Date of filing: 28.02.2022
(51) Int. Cl.: A61B 5/0245, A61B 5/11

(54) **ELECTRONIC DEVICE, METHOD FOR CONTROLLING ELECTRONIC DEVICE, AND PROGRAM**

(30) Priority: 25.03.2021 JP 2021051401
(71) Applicant: Casio Computer Co., Ltd., Tokyo 151-8543 (JP)
(72) Inventor: HIKOSAKA, Naotaka, Hamura-shi, Tokyo 205-8555 (JP)
(74) Representative: Plougmann Vingtoft a/s
(86) International application number: PCT/JP2022/008226
(87) International publication number: WO 2022/202121

(57) **Abstract**

An electronic device to be fitted on a user, provided with a light-emitting part for emitting light on the user's skin, a light-receiving part provided at a position where light reflected by the user's skin can be received when the device is fitted on the user and the light-emitting part is emitting light, and a control part. The control part performs a skin assessment for determining whether or not the user's skin fulfills a prescribed darkness condition on the basis of the amount of light received by the light-receiving part and performs a fitting assessment for determining whether or not the device is fitted on the user at least on the basis of the amount of light received by the light-receiving part, if it is determined in the skin assessment that the darkness condition is fulfilled, the fitting assessment is not performed.

## Description

### TECHNICAL FIELD

The present invention pertains to an electronic device, a method for controlling an electronic device, and a program.

### BACKGROUND ART

Heretofore, a technique for, in an electronic device that a user wears and uses, using a light-receiver to receive reflected light of light emitted onto the user's skin and detecting a pulse on the basis of change in the quantity of received light is known (for example, Patent Literature 1). In addition, there is a known technique that, making use of the fact that the quantity of light received by the light-receiver decreases when the electronic device is not being worn by a user, determines whether the electronic device is being worn by a user on the basis of the magnitude of the quantity of light received.

### CITATION LIST

### PATENT DOCUMENTS

Patent Document 1: Japanese Patent No. 4476664

### SUMMARY OF INVENTION

### Technical Problem

However, the quantity of light incident on the light-receiver (in other words the quantity of light received by the light-receiver) after reflecting from a user's skin when the electronic device is worn by a user decreases the darker the skin. Accordingly, there is a problem that, even if the electronic device is being worn by a user, due to the darkness of the user's skin, there may be a misdetermination that the electronic device is not being worn due to the quantity of received light decreasing.

An objective of this invention is to provide an electronic device, a method for controlling an electronic device, and a program for suitably performing a determination pertaining to a worn state.

### Solution to Problem

In order to solve the above-described problem, an electronic device according to the present invention is
an electronic device that is configured to be worn by a user and is provided with:
a light-emitter that emits light onto skin of the user;
a light-receiver provided at a position for enabling reception of the light reflected by the skin of the user when the electronic device is being worn by the user and the light-emitter is emitting the light; and
a controller, wherein
the controller:
   performs a skin determination for, on the basis of a quantity of light received by the light-receiver, determining whether the skin of the user satisfies a predetermined darkness condition;
   performs a being-worn determination for, on the basis of at least the quantity of light received by the light-receiver, determining whether the user is wearing the electronic device; and
   does not perform the being-worn determination in response to determining in the skin determination that the darkness condition is satisfied.

In order to solve the above-described problem, a method for controlling an electronic device according to the present invention is
a method for controlling an electronic device that is configured to be worn by a user and is provided with a light-emitter that emits light onto skin of the user and a light-receiver provided at a position for enabling reception of the light reflected by the skin of the user when the electronic device is being worn by the user and the light-emitter is emitting the light, the method including:
a skin determination step for determining, on the basis of a quantity of light received by the light-receiver, whether the skin of the user satisfies a predetermined darkness condition, and
a being-worn determination step for determining, on the basis of at least the quantity of light received by the light-receiver, whether the user is wearing the electronic device,
the being-worn determination step not being performed in response to determining in the skin determination step that the darkness condition is satisfied.

In order to solve the above-described problem, a program according to the present invention is
a program executed by a computer provided in an electronic device that is configured to be worn by a user and is provided with a light-emitter that emits light onto skin of the user and a light-receiver provided at a position for enabling reception of the light reflected by the skin of the user when the electronic device is being worn by the user and the light-emitter is emitting the light, the program causing the computer to function as a controller, wherein
the controller:
   performs a skin determination for, on the basis of a quantity of light received by the light-receiver, determining whether the skin of the user satisfies a predetermined darkness condition,
   performs a being-worn determination for, on the basis of at least the quantity of light received by the light-receiver, determining whether the user is wearing the electronic device, and
   does not perform the being-worn determination in response to determining in the skin determination that the darkness condition is satisfied.

### Advantageous Effects of Invention

By virtue of the present invention, it is possible to suitably perform a determination pertaining to a worn state.

### BRIEF DESCRIPTION OF DRAWINGS

[FIG. 1] This is a perspective view that illustrates an appearance of an electronic timepiece.
[FIG. 2] This is a side surface view of the electronic timepiece.
[FIG. 3] This is a block view that illustrates a functional configuration of the electronic timepiece.
[FIG. 4A] This is a view for illustrating an example of a difference in quantities of received light due to skin darkness.
[FIG. 4B] This is a view for illustrating an example of a difference in quantities of received light due skin darkness.
[FIG. 5] This is a flow chart that illustrates a control procedure for a pulse rate measurement process.
[FIG. 6] This is a flow chart that illustrates a control procedure for a pulse rate measurement start process.
[FIG. 7] This is a flow chart that illustrates a control procedure for a skin determination process.
[FIG. 8] This is a flow chart that illustrates a control procedure for a being-worn determination process.
[FIG. 9] This is a flow chart that illustrates a control procedure for a pulse rate measurement resumption process.

### DESCRIPTION OF EMBODIMENTS

With reference to the drawings, description is given below regarding embodiments for an electronic device, a method for controlling an electronic device, and a program that are according to the present invention.

### (Configuration of electronic timepiece)

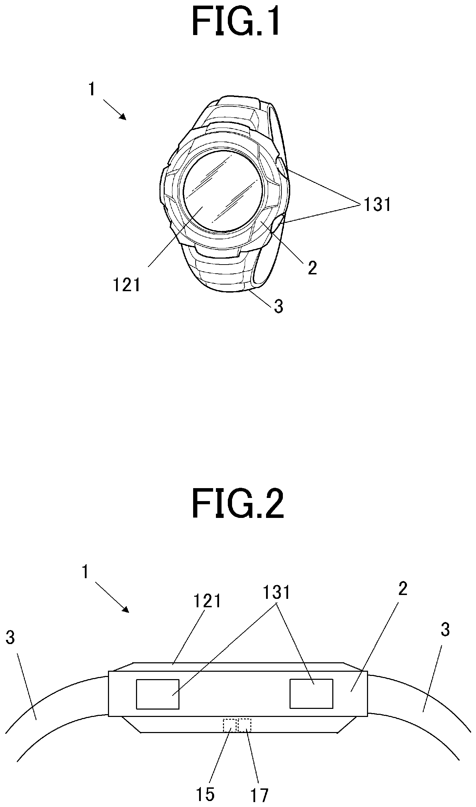

FIG. 1 is a perspective view that illustrates an appearance of an electronic timepiece 1.

The electronic timepiece 1 (an electronic device) is a wristwatch that is used while being worn on a user's wrist. The electronic timepiece 1 is provided with a body 2 on which, inter alia, a display screen 121 and an operation button 131 are provided, and a belt 3 that is attached to the body 2. The display screen 121 performs a digital display in a dot-matrix format. In the display screen 121, the electronic timepiece 1 displays, inter alia, a measurement result for the user's pulse rate (heart rate), in addition to basic information such as the time and date.

FIG. 2 is a side surface view of the electronic timepiece 1.

The electronic timepiece 1 is provided with, inside the body 2, a light-emitter 15 and a light-receiver 17 which are used to detect a pulse. The light-emitter 15 and the light-receiver 17 are provided on the back surface of the body 2, in other words near the surface which comes into contact with the user's wrist when worn. The light-emitter 15 emits light outward from the back surface of the body 2. When the electronic timepiece 1 is being worn by the user, light emitted from the light-emitter 15 is reflected by the skin of the user's wrist. The light-receiver 17 is provided at a position that enables reception of this light that was reflected by the user's skin. Some light irradiated on the user's skin is absorbed by blood within blood vessels. Accordingly, the quantity of light received by the light-receiver 17 from among the light reflected from the skin changes over time in response to change of a blood flow rate accompanying pulsation by the heart. A pulse is detected on the basis of change in this quantity of received light, and a pulse rate is measured on the basis of this detected pulse.

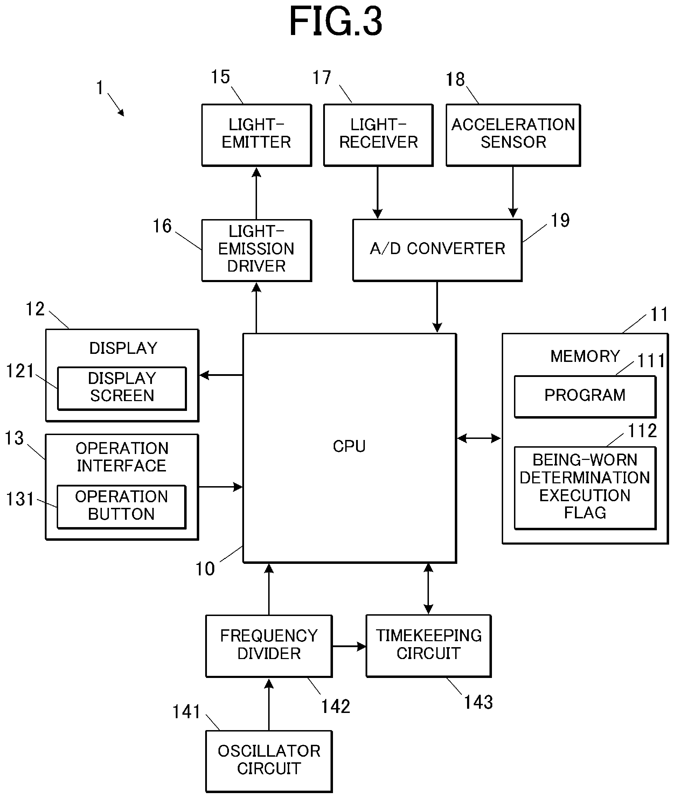

FIG. 3 is a block view that illustrates a functional configuration of the electronic timepiece 1.

The electronic timepiece 1 is provided with, inter alia, a CPU 10 (Central Processing Unit), a memory 11 (a storage), a display 12, an operation interface 13, an oscillator circuit 141, a frequency divider 142, a timekeeping circuit 143, the light-emitter 15, a light-emission driver 16, the light-receiver 17, an acceleration sensor 18, and an A/D converter 19.

The CPU 10 is a processor that performs various arithmetic processing, and comprehensively controls operation by each unit in the electronic timepiece 1. The CPU 10 reads out and executes a program 111 stored in the memory 11 to thereby function as a controller (control means) that performs various control operations.

For example, the CPU 10 causes the display 12 to display a datetime counted by the timekeeping circuit 143. In addition, the CPU 10 detects a pulse on the basis of change in the quantity of light received by the light-receiver 17, and causes the display 12 to display a measurement result for a pulse rate (number of pulses per minute). The CPU 10 also performs a skin determination for, on the basis of the quantity of light received by the light-receiver 17, determining whether the user's skin satisfies a predetermined darkness condition. In addition, the CPU 10, on the basis of the quantity of light received by the light-receiver 17 and a result of detecting acceleration by the acceleration sensor 18, performs a being-worn determination to determine whether the electronic timepiece 1 (the host device) is being worn by a user. The skin determination and the being-worn determination are described in detail below.

The memory 11 provides a work memory space to the CPU 10, and stores various data. For example, the memory 11 includes a RAM (Random-Access Memory) and a non-volatile memory. The RAM is used for arithmetic processing by the CPU 10, and also stores temporary data. The non-volatile memory is a non-transitory recording medium that can be read by the CPU 10, which corresponds to a computer. The non-volatile memory is a flash memory, for example, and stores various types of data in addition to the program 111. The program 111 includes a control program for controlling basic operation by the electronic timepiece 1, as well as, inter alia, an application program (hereinafter, written as "pulse rate measurement application") for measuring a pulse rate and causing the display 12 to display a result. Data stored in the memory 11 includes a being-worn determination execution flag 112. The being-worn determination execution flag 112 is used to determine whether to execute a being-worn determination process which is described below. The being-worn determination execution flag 112 is one-bit data, for example.

The display 12 is provided with the display screen 121, and performs a digital display on the display screen 121 under control by the CPU 10. Here, the display screen 121 is capable of displaying in a dot-matrix format, and is a liquid-crystal display screen, for example.

The operation interface 13 is provided with a plurality of operation buttons 131. The operation interface 13 accepts an input operation (for example, a press operation) by a user with respect to an operation button 131, and outputs the input operation as an input signal to the CPU 10. The CPU 10 executes a process corresponding to a function for the operation button 131 to which the input operation was performed. A function allocated to each operation button 131 may be switched in response to an operation mode for the electronic timepiece 1. An operation button 131 may include a winding crown. In addition, the operation interface 13 may have a touch panel that is overlappingly provided on the display screen 121.

The oscillator circuit 141 generates a clock signal that has a predetermined oscillation frequency, and outputs the clock signal to the frequency divider 142. The frequency divider 142 divides the clock signal inputted from the oscillator circuit 141 to thereby converts the clock signal into a frequency that is necessary for operation by each unit in the electronic timepiece 1, and outputs the frequency. An output destination for a signal divided by the frequency divider 142 includes the timekeeping circuit 143.

The timekeeping circuit 143 counts a signal that has a predetermined frequency and is inputted from the frequency divider 142 to thereby count the current datetime, and holds the current datetime. A format of a datetime held by the timekeeping circuit 143 is not limited to being represented by year, month, day, hour, minute, and second, and may be a format that is suitable and appropriate for processing by, inter alia, the CPU 10.

The light-emitter 15 is provided with a light-emitting element that emits light, such as a LED (Light-Emitting Diode). The light-emitter 15 according to the present embodiment is provided with an LED that emits green light, which is easily absorbed by hemoglobin in blood, for example light for which a peak wavelength is 520-530 nm. The LED in the light-emitter 15 emits light in response to a drive current supplied from the light-emission driver 16.

The light-emission driver 16 controls output of the drive current to the light-emitter 15 in accordance with a control signal from the CPU 10 to thereby cause the LED in the light-emitter 15 to emit light or stop emitting light.

The light-receiver 17 is provided with a light-receiving element that detects light and outputs an electrical signal which corresponds to a quantity of received light. Here, the quantity of received light is the intensity of incident light, for example. For example, it is possible to use, inter alia, a photodiode or an illuminance sensor as the light-receiving element.

The acceleration sensor 18 detects acceleration by the electronic timepiece 1 arising in response to, inter alia, motion by the user, and outputs an electrical signal which corresponds to the acceleration. The acceleration sensor 18 detects acceleration for each of the three axial directions in a Cartesian coordinate system, for example.

The A/D converter 19 converts the electrical signals outputted from the light-receiver 17 and the acceleration sensor 18 to digital data, and outputs the digital data to the CPU 10. Accordingly, digital data representing the quantity of light received by the light-receiver 17 is outputted from the A/D converter 19 to the CPU 10. In addition, digital data representing a result of detecting acceleration by the acceleration sensor 18 is outputted from the A/D converter 19 to the CPU 10. The A/D converter 19 may be separately provided for each of the light-receiver 17 and the acceleration sensor 18.

### (Operation by electronic timepiece)

Next, regarding operation by the electronic timepiece 1, description is mainly given for an operation for measurement of a pulse rate.

As described above, measurement of a pulse rate (and therefore detection of a pulse) in the electronic timepiece 1 accompanies emission of light by the LED in the light-emitter 15 (simply written as emission of light by the light-emitter 15 below). Accordingly, in a case where the electronic timepiece 1 is removed from the user's wrist during measurement of the pulse rate, causing the light-emitter 15 to stop emitting light to thereby stop measurement of the pulse rate is desirable in terms of appearance as well as the perspective of power consumption.

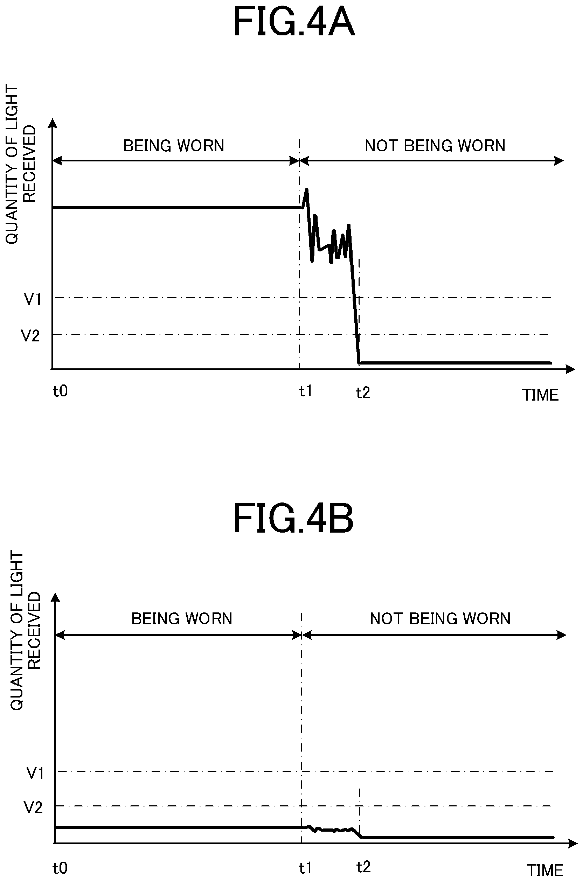

Accordingly, the electronic timepiece 1 according to the present embodiment performs a being-worn determination for determining whether the host device is being worn by a user after measurement of a pulse rate is started. This being-worn determination is performed on the basis of whether the quantity of light received from the light-receiver 17 is greater than a predetermined reference value (a second reference value V2 illustrated in FIG. 4A and FIG. 4B). In a case where the electronic timepiece 1 is being worn on a user's wrist, light emitted from the light-emitter 15 is reflected by skin and is incident on the light-receiver 17. In contrast, in a case where the electronic timepiece 1 has been removed from the user's wrist, light emitted from the light-emitter 15 diffuses without being reflected by skin and for the most part does not return to the light-receiver 17. Accordingly, in a case where the electronic timepiece 1 is not being worn by the user, the quantity of light received by the light-receiver 17 greatly decreases in comparison to a case where the electronic timepiece 1 is being worn. Therefore, it is possible to determine whether the electronic timepiece 1 is being worn by a user, on the basis of whether the quantity of light received by the light-receiver 17 is greater than the second reference value V2. The second reference value V2 is set to a value between a quantity of light received for when the electronic timepiece 1 is being worn by a user, and a quantity of light received for when the electronic timepiece 1 is not being worn by a user. When it is determined that the electronic timepiece 1 is not being worn by a user, measurement of a pulse rate is stopped.

However, the ratio of light that is reflected by the user's skin and is incident on the light-receiver 17 with respect to light emitted from the light-emitter 15 decreases the darker the skin (for example, the deeper the skin color) . Accordingly, the quantity of light received by the light-receiver 17 greatly differs in accordance with the darkness of the skin of a user wearing the electronic timepiece 1. Accordingly, even if the electronic timepiece 1 is being worn by a user, due to the darkness of their skin, there may be a misdetermination that the electronic timepiece 1 is not being worn due to the quantity of received light decreasing. When such a misdetermination arises, measurement of a pulse rate will unfortunately stop at an unintended timing while the electronic timepiece 1 is being worn. The darkness of skin includes, inter alia, darkness caused by a diffusion state or reflectance of light changing due to a cosmetic product or the like being applied to the skin, in addition to darkness due to the color of bare skin.

FIG. 4A and FIG. 4B are views for illustrating an example of a difference in quantities of received light due to the darkness of skin.

FIG. 4A illustrates an example of a quantity of light received by the light-receiver 17 in a case where a user having light skin has used the electronic timepiece 1. FIG. 4B illustrates an example of a quantity of light received by the light-receiver 17 in a case where a user having dark skin has used the electronic timepiece 1. In more detail, FIG. 4A illustrates a case where a user having skin classified as type I from among Fitzpatrick skin types has used the electronic timepiece 1. In contrast, FIG. 4B illustrates a case where a user having skin classified as type VI from among Fitzpatrick skin types has used the electronic timepiece 1.

In FIG. 4A and FIG. 4B, the electronic timepiece 1 is being worn by a user in a duration from a timing t0 to a timing t1. In addition, the electronic timepiece 1 is removed from the user's wrist at the timing t1, and the electronic timepiece 1 is left alone on a table at a timing t2.

In such a situation, in a case of light skin as illustrated in FIG. 4A, the quantity of received light is greater than the second reference value V2 while the electronic timepiece 1 is being worn (timing t0 to t1) and thus an appropriate determination that the electronic timepiece 1 is being worn is made. In addition, in the state where the electronic timepiece 1 has been removed and is left alone (timing t2 and thereafter), the quantity of received light becomes less than or equal to the second reference value V2, and thus an appropriate determination that the electronic timepiece 1 is not being worn is made.

In contrast, in a case of dark skin as illustrated in FIG. 4B, the quantity of received light is less than or equal to the second reference value V2 even while the electronic timepiece 1 is being worn (timing t0 to t1), and thus an inappropriate determination that the electronic timepiece 1 is not being worn is made despite the user wearing the electronic timepiece 1.

Accordingly, the electronic timepiece 1 according to the present embodiment performs a skin determination for determining whether the user's skin satisfies a predetermined darkness condition, on the basis of the quantity of light received by the light-receiver 17 while the electronic timepiece 1 is being worn. In a case where it is determined that the darkness condition is not satisfied (in other words, in a case where it is determined that there is light skin), the being-worn determination is performed. In contrast, in a case where it is determined that the darkness condition is satisfied (in other words, in a case where it is determined that there is dark skin), the being-worn determination is not performed. As a result, the occurrence of a defect where, due to skin darkness, a misdetermination that the electronic timepiece 1 is not being worn is made despite the electronic timepiece 1 being worn is suppressed. A user's skin that is a target of a skin determination may be bare skin, or may be skin in a state where a cosmetic product or the like has been applied.

The skin determination determines that the darkness condition is satisfied in a case where the quantity of light received by the light-receiver 17 is less than or equal to a first reference value V1. Here, it is desirable for the first reference value V1 is to be greater than the second reference value V2 as illustrated in FIG. 4A and FIG. 4B. The quantity of light received by the light-receiver 17 while the electronic timepiece 1 is being worn can fluctuate due to, inter alia, the brightness of the environment or the manner in which the electronic timepiece 1 is worn, even if there is the same user. Accordingly, when the second reference value V2 is used to perform a skin determination, it may be that the quantity of received light for when a being-worn determination is performed becomes less than or equal to the second reference value V2 and a misdetermination that the electronic timepiece 1 is not being worn arises, even if it is determined that the darkness condition is not satisfied (in other words, there is light skin). It is possible to reduce such misdeterminations by using the first reference value V1, which results from adding a predetermined margin to the second reference value V2, to perform a skin determination.

A method for the skin determination can be rephrased as follows. In other words, the skin determination determines, on the basis of the quantity of light received by the light-receiver 17 when the electronic timepiece 1 is being worn, whether the skin of a user satisfies a predetermined lightness condition. In a case where it is determined that the lightness condition is satisfied (in other words, in a case where it is determined that there is light skin), the being-worn determination is performed. In contrast, in a case where it is determined that the lightness condition is not satisfied (in other words, in a case where it is determined that there is dark skin), the being-worn determination is not performed. The skin determination in this case determines that the lightness condition is satisfied in a case where the quantity of light received by the light-receiver 17 is greater than the first reference value V1.

Note that, even in a case where the darkness condition is satisfied and the quantity of received light becomes less than the second reference value V2 even while the electronic timepiece 1 is being worn as in FIG. 4B, fluctuation of the quantity of received light that correspond to change of the blood flow rate is detected, and thus detection of a pulse and measurement of a pulse rate are possible.

Next, description is given regarding a pulse rate measurement process for causing the electronic timepiece 1 to perform a pulse rate measurement operation that includes the above-described skin determination and being-worn determination.

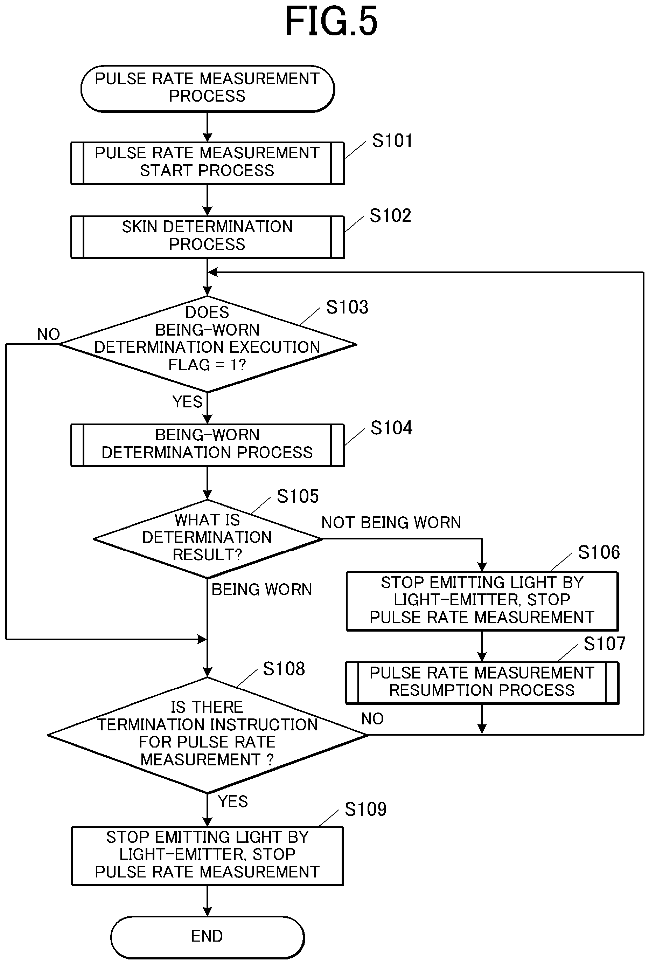

FIG. 5 is a flow chart that illustrates a procedure by which the CPU 10 controls a pulse rate measurement process.

When the pulse rate measurement process is started, the CPU 10 executes a pulse rate measurement start process (step S101).

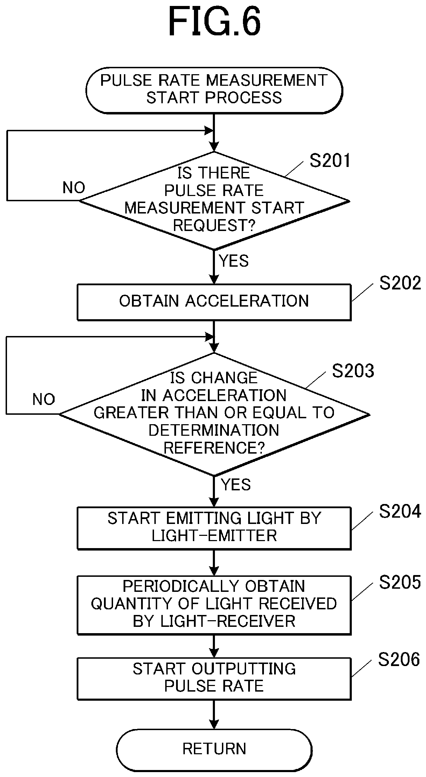

FIG. 6 is a flow chart that illustrates a procedure by which the CPU 10 controls the pulse rate measurement start process.

When the pulse rate measurement start process is started, the CPU 10 determines whether there is a pulse rate measurement start request (step S201). For example, in a case where a user input operation for causing pulse rate measurement to start is accepted on the pulse rate measurement application, it is determined that there is a pulse rate measurement start request. In a case where it is determined that there is no pulse rate measurement start request ("NO" in step S201), the CPU 10 re-executes the processing in step S201.

In a case where it is determined that there is a pulse rate measurement start request ("YES" in step S201), the CPU 10 obtains a result of detecting acceleration by the acceleration sensor 18 (step S202).

For the obtained result of detecting acceleration, the CPU 10 determines whether change in acceleration is greater than or equal to a predetermined determination reference (step S203). If the change in acceleration is greater than or equal to the determination reference, it is deemed that the user is wearing the electronic timepiece 1, and processing pertaining to pulse rate measurement is subsequently advanced to. The determination reference is defined to facilitate the acceleration becoming greater than or equal to the determination reference when a user is wearing the electronic timepiece 1. In other words, the determination reference is defined to be near a lower limit from among a range of change in acceleration that arises in response to movement by the user while the user is wearing the electronic timepiece 1. In a case where it is determined that change in acceleration is less than the determination reference ("NO" in step S203), it is possible to determine that the electronic timepiece 1 is not being worn by a user and causing pulse rate measurement to start is not appropriate, and thus the CPU 10 executes the processing step S203 again without proceeding to the next step.

In a case where it is determined that change in acceleration is greater than or equal to the determination reference ("YES" in step S203), the CPU 10 starts measuring a pulse rate. In other words, the light-emitter 15 is caused to emit light (step S204), a result of detecting a quantity of light received by the light-receiver 17 is periodically obtained (step S205), and a pulse is detected from change in the obtained quantity of light received. A frequency of obtaining the quantity of light received by the light-receiver 17 is not particularly limited, but can be set to every 10 msec, for example. The CPU 10 calculates a pulse rate on the basis of a result of detecting a pulse, and outputs an obtained pulse rate (causes a display on the display 12) (step S206).

When step S206 ends, the CPU 10 causes the pulse rate measurement start process to end, and returns the processing to the pulse rate measurement process in FIG. 5.

In FIG. 5, when the pulse rate measurement start process (step S101) ends, the CPU 10 executes a skin determination process (step S102: skin determination step).

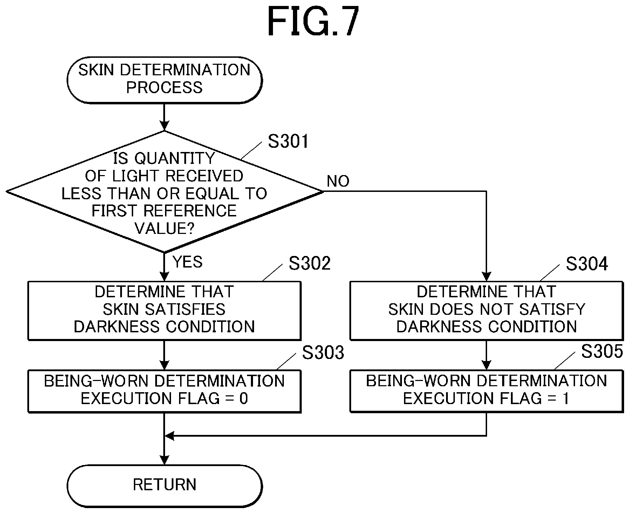

FIG. 7 is a flow chart that illustrates a procedure by which the CPU 10 controls the skin determination process.

When the skin determination process is started, the CPU 10 determines whether the quantity of light received by the light-receiver 17 is less than or equal to the first reference value V1 (step S301). Here, it may be that the determination is performed using any one item of data from among quantities of light that are received by the light-receiver 17 and are obtained in order to detect a pulse.

In a case where it is determined that the quantity of light received is less than or equal to the first reference value V1 ("YES" in step S301), the CPU 10 determines that skin satisfies the darkness condition (step S302) and sets a value for a being-worn determination execution flag to "0" (step S303).

In a case where it is determined that the quantity of light received is greater than the first reference value V1 ("NO" in step S301), the CPU 10 determines that skin does not satisfy the darkness condition (step S304) and sets a value for the being-worn determination execution flag to "1" (step S305) .

This skin determination process is performed in a case where it is determined that the change in the acceleration is greater than or equal to the determination reference ("YES" in step S203) and pulse rate measurement (step S206) has started, in the pulse rate measurement start process in FIG. 6. In other words, the skin determination is performed on the basis of the quantity of light received by the light-receiver 17 when a pulse is being detected. As a result, it is possible to appropriately perform a skin determination on the basis of a quantity of light received that reflects skin lightness, in a state where the electronic timepiece 1 is being worn by a user.

When step S303 or S304 ends, the CPU 10 causes the skin determination process to end, and returns the processing to the pulse rate measurement process in FIG. 5.

In FIG. 5, when the skin determination process (step S102) ends, the CPU 10 determines whether the being-worn determination execution flag is "1" (step S103). In a case where it is determined that the being-worn determination execution flag is "1", ("YES" in step S103), the CPU 10 executes a being-worn determination process (step S104: being-worn determination step).

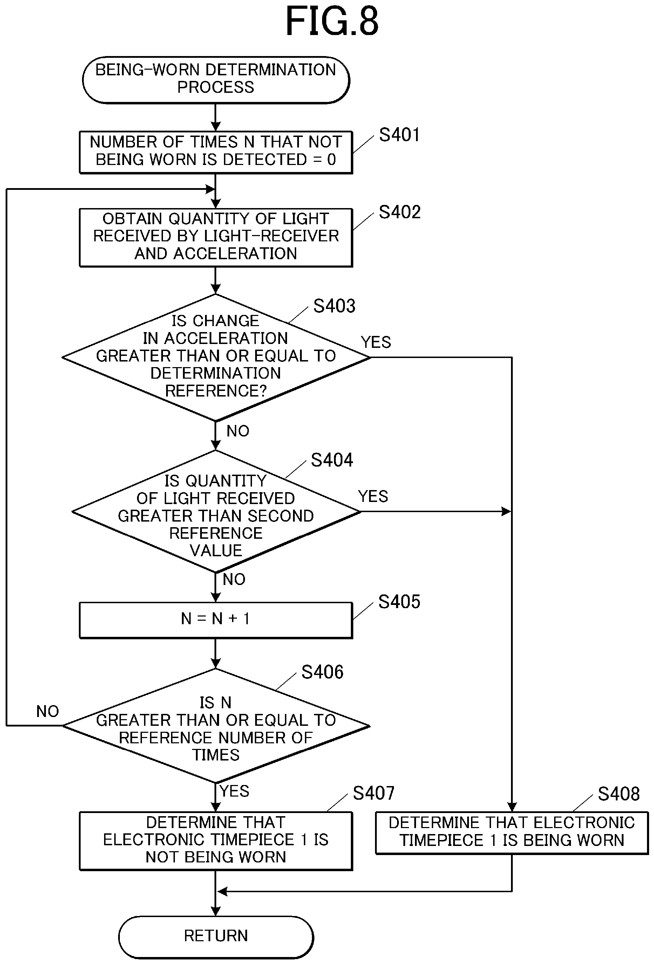

FIG. 8 is a flow chart that illustrates a procedure by which the CPU 10 controls the being-worn determination process.

When the being-worn determination process is started the CPU 10 assigns "0" to a variable N representing a number of times that the electronic timepiece not being worn is detected (step S401).

The CPU 10 obtains a result of detecting a quantity of light received by the light-receiver 17 and a result of detecting acceleration by the acceleration sensor 18 (step S402). The CPU 10 determines whether change in acceleration is greater than or equal to the aforementioned determination reference, for the obtained result of detecting acceleration (step S403).

In a case where it is determined that the change in acceleration is less than the determination reference ("NO" in step S403), the CPU 10 determines whether the quantity of light received by the light-receiver 17 is greater than the second reference value V2 (step S404). In a case where it is determined here that the quantity of light received is less than or equal to the second reference value V2 ("NO" in step S404), it is possible that the electronic timepiece 1 is not being worn by a user because a state has been entered in which the change in acceleration is less than the determination reference and the quantity of light received is less than or equal to the second reference value V2 for a being-worn determination. Accordingly, the CPU 10 increments the variable N representing the number of times that the electronic timepiece 1 not being worn is detected (step S405) .

The CPU 10 determines whether the variable N is greater than or equal to a reference number of times (step S406) . In a case where it is determined that the variable N is greater than or equal to the reference number of times ("NO" in step S406), the CPU 10 returns the processing to step S402, and re-executes the processing loop of steps S402 through S406. In a case where it is determined that the variable N is greater than or equal to the reference number of times ("YES" in step S406), the CPU 10 determines that the user is not wearing the electronic timepiece 1 (step S407). In this manner, in a case where the processing loop of steps S402 through S406 is repeated until the variable N becomes the reference number of times - in other words in a case where it continues to be determined in the processing loop that there is the possibility that the electronic timepiece 1 is not being worn for a predetermined amount of time - step S407 is advanced to and it is determined that the electronic timepiece 1 is not being worn. The reference number of times is defined such that an amount of time required for the variable N to become the reference number of times becomes the predetermined amount of time described above. The predetermined amount of time can be discretionarily set, and can be set to approximately five seconds, for example.

In contrast, in a case where it is determined in step S403 that the change in acceleration is greater than or equal to the determination reference ("YES" in step S403) or in a case where it is determined in step S404 that the quantity of light received is greater than the second reference value V2 ("YES" in step S404), the CPU 10 determines that the electronic timepiece 1 is being worn by a user (step S408) . By virtue of this determination method, for example, even in a case where a user who is wearing the electronic timepiece 1 causes their arm to be stationary, it is possible to appropriately determine that a state has been entered in which the user is wearing the electronic timepiece 1, on the basis of the quantity of light received by the light-receiver 17 being greater than or equal to the second reference value V2. Accordingly, a being-worn determination that is more accurate than a determination method using only a detection result by the acceleration sensor 18 is possible.

When step S407 or step S408 ends, the CPU 10 causes the being-worn determination process to end, and returns the processing to the pulse rate measurement process in FIG. 5.

In FIG. 5, when the being-worn determination process (step S104) ends, the CPU 10 switches subsequent processing in response to a result of the being-worn determination (step S105). In a case where the determination result is "electronic timepiece 1 not being worn", the CPU 10 causes the light-emitter 15 to stop emitting light, and causes pulse detection and pulse rate measurement to stop (step S106). Subsequently, the CPU 10 executes a pulse rate measurement resumption process for resuming pulse rate measurement at an appropriate timing when the electronic timepiece 1 is being worn again.

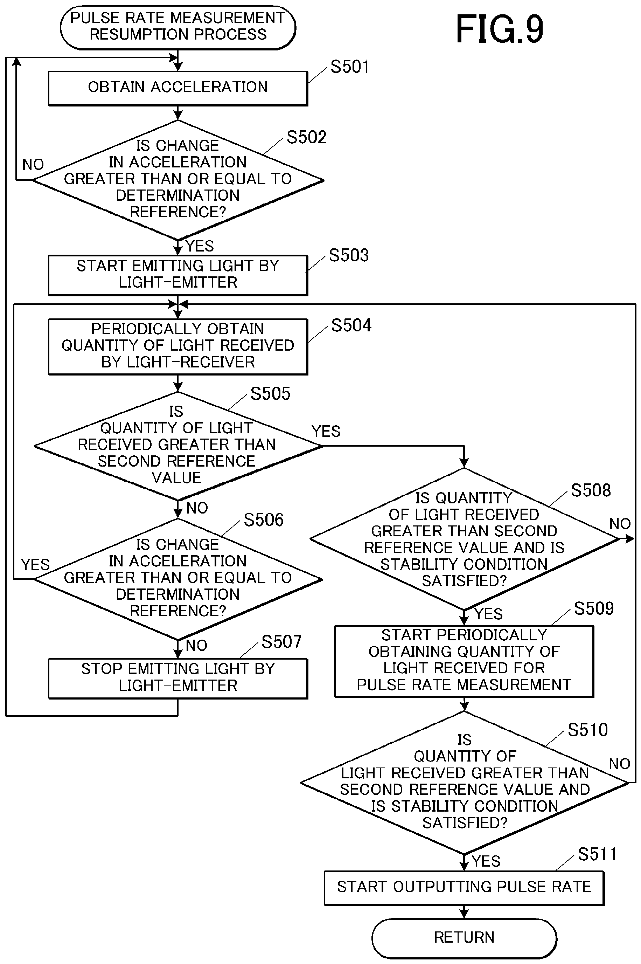

FIG. 9 is a flow chart that illustrates a procedure by which the CPU 10 controls the pulse rate measurement resumption process.

When the pulse rate measurement resumption process is started, the CPU 10 obtains a result of detecting acceleration by the acceleration sensor 18 (step S501). For the obtained result of detecting acceleration, the CPU 10 determines whether change in acceleration is greater than or equal to the above-described determination reference (step S502) and, in a case of determining that change in acceleration is less than the determination reference ("NO" in step S502), returns the processing to step S501.

In a case where it is determined that change in acceleration is greater than or equal to the determination reference ("YES" in step S502), the CPU 10 causes the light-emitter 15 to emit light (step S503), and periodically obtains a result of detecting the quantity of light received by the light-receiver 17 (step S504). A frequency of obtaining the quantity of light received here may be set to less than a detection frequency that is for when detecting a pulse, and may be step to approximately several tens of msec, for example.

The CPU 10 determines whether the quantity of light received by the light-receiver 17 is greater than the second reference value V2 (step S505). In a case where it is determined that the quantity of light received is less than or equal to the second reference value V2 ("NO" in step S505), the CPU 10 obtains a result of detecting acceleration by the acceleration sensor 18 again, and determines whether change in acceleration is greater than or equal to the determination reference (step S506) . In a case where it is determined that change in acceleration is less than the reference value ("NO" in step S506), it is possible to determine at this stage that a user is not wearing the electronic timepiece 1 and resuming pulse rate measurement is not appropriate. Accordingly, the CPU 10 causes the light-emitter 15 to stop emitting light (step S507), and returns the processing to step S501.

In contrast, in a case where it is determined in step S506 that change in acceleration is greater than or equal to the determination reference ("YES" in step S506), the CPU 10 returns the processing to step S504, and re-executes the processing loop of step S504 through step S506. In this processing loop, in a case where it is determined in step S505 that the quantity of light received is greater than the second reference value V2 ("YES" in step S505), it is possible to determine that it is possible for a user to be wearing the electronic timepiece 1. Accordingly, the CPU 10 also confirms that a state in which the electronic timepiece is 1 being worn by a user has been entered, and then executes processing in steps S508 through 511 for resuming pulse rate measurement.

Firstly, the CPU 10 determines whether the quantity of light received by the light-receiver 17 is greater than the second reference value V2 and whether a predetermined stability condition is satisfied (step S508). Here, the stability condition is determined by the following method, for example. In other words, from among two quantities of light received that were obtained at the two most recent obtainment timings, letting the lesser one be R1 and the greater one be R2, it is determined that the stability condition is satisfied in a case where (R2 - R1)/R1 is less than a predetermined upper limit. The abovementioned upper limit can be set, as appropriate, but the smaller the upper limit, the more it is possible to reduce the occurrence of a defect in which pulse rate measurement is resumed in a not-worn state. Note that a method of determining the stability condition is not limited to that described above.

In a case where it is determined that the quantity of light received is greater than the second reference value V2 and the stability condition is satisfied ("YES" in step S508), the CPU 10 starts periodically obtaining a quantity of light received for pulse rate measurement (step S509). Here, it may be that the quantity of light received is obtained at a higher frequency (for example, every 10 msec) than the obtainment frequency in step S504. The CPU 10 starts pulse detection on the basis of the obtained quantity of light received.

The CPU 10 again determines whether the quantity of light received by the light-receiver 17 is greater than the second reference value V2 and whether a predetermined stability condition is satisfied (step S510). In a case where it is determined in step S508 or step S509 that the quantity of light received is less than or equal to the second reference value V2 or the quantity of light received does not satisfy the stability condition ("NO" in step S508 or step S510), the CPU 10 returns to the processing step S504.

In a case where it is determined in step S510 that the quantity of light received is greater than the second reference value V2 and the stability condition is satisfied ("YES" in step S510), the CPU 10 measures a pulse rate on the basis of, inter alia, a pulse detection frequency, and outputs the obtained pulse rate to thereby cause the display 12 to display the obtained pulse rate (step S511). In other words, measurement of the pulse rate resumes. A similar determination to that in step S508 is performed again in step S510 in order to cause pulse rate measurement to resume after confirming that the user has not removed the electronic timepiece 1 in the duration from step S508 to step S510.

When step S511 ends, the CPU 10 causes the pulse rate measurement resumption process to end, and returns the processing to the pulse rate measurement process in FIG. 5.

In FIG. 5, when the pulse rate measurement resumption process (step S107) ends, the CPU 10 returns the processing to step S103.

In a case where it is determined in step S103 that the being-worn determination execution flag is "0" ("NO" in step S103), the CPU 10 causes the processing to transition to step S108 without executing the processing in steps S104 through 107. In addition, in a case where it is determined in step S105 that the determination result of the being-worn determination process is "electronic timepiece 1 being worn", the CPU 10 causes the processing to transition to step S108.

In step S108, the CPU 10 determines whether an instruction for terminating pulse rate measurement has been performed. For example, in a case where a user input operation for terminating pulse rate measurement is accepted on the pulse rate measurement application, it is determined that a pulse rate measurement termination instruction has been performed. In a case where it is determined that a termination instruction has not been performed ("NO" in step S108), the CPU 10 returns the processing to step S103. In a case where it is determined that a termination instruction has been performed ("YES" in step S108), the CPU 10 causes the light-emitter 15 to stop emitting light and thereby cause pulse detection and pulse rate measurement to stop (step S109), and causes the pulse rate measurement process to end.

### (Effect)

As above, the electronic timepiece 1, which corresponds to an electronic device worn by a user and is according to the present embodiment, is provided with: the light-emitter 15 which emits light onto the user's skin; the light-receiver 17 which is provided at a position that enables the reception of light reflected by the user's skin when the user is wearing the host device and when the light-emitter 15 is emitting light; and the CPU 10 which functions as a controller. The CPU 10, which corresponds to a controller, also performs a skin determination for, on the basis of the quantity of light received by the light-receiver 17, determining whether the user's skin satisfies a predetermined darkness condition. In addition, the CPU 10, which corresponds to a controller, performs the being-worn determination for determining whether the host device is being worn by the user on the basis of at least a quantity of light received by the light-receiver 17, and does not perform the being-worn determination in a case of determining that a darkness condition is satisfied in the skin determination. As a result, it is possible to suitably perform a determination pertaining to a worn state. For example, it is possible to make it less likely for a defect to arise in which a misdetermination that the electronic timepiece 1 is not being worn is made when a user is wearing the electronic timepiece 1.

In addition, the CPU 10, which corresponds to a controller, performs a being-worn determination in a case of determining that the darkness condition is not satisfied in the skin determination. As a result, because a being-worn determination is performed in the case of light skin, it is possible to make it less likely for a defect in which a misdetermination that the electronic timepiece 1 is not being worn being made due to the quantity of light received for light reflected by skin being small.

In addition, the CPU 10, which corresponds to a controller, detects a pulse on the basis of change in the quantity of light received by the light-receiver 17. With such a configuration, it is possible to perform a skin determination using the light-emitter 15 and the light-receiver 17 which are for pulse detection. Accordingly, it is possible to make it less likely for a misdetermination to arise due to performing a skin determination, while avoiding complicating the configuration of the electronic timepiece 1 and raising manufacturing costs.

In addition, the CPU 10, which corresponds to a controller, performs the skin determination on the basis of the quantity of light received by the light-receiver 17 when a pulse is being detected. As a result, it is possible to appropriately perform a skin determination on the basis of a quantity of light received that reflects skin lightness, in a state where the electronic timepiece 1 is being worn by a user.

In addition, in the skin determination, the CPU 10 which corresponds to a controller determines that the darkness condition is satisfied in a case where the quantity of light received by the light-receiver 17 is less than or equal to a first reference value V1. As a result, it is possible to perform the skin determination by a simple process.

In addition, in a case where the quantity of light received by the light-receiver 17 is greater than the second reference value V2 in a being-worn determination, the CPU 10 which corresponds to a controller determines that the host device is being worn by a user, and the first reference value V1 is greater than the second reference value V2. In this manner, the first reference value V1, which is greater than the second reference value V2 used in the being-worn determination, is used to perform a skin determination, whereby it is possible to prevent a misdetermination that the not-worn state has been entered from being made immediately even if the quantity of light received in a being-worn determination becomes less than the first reference value V1 due to, inter alia, the brightness of the environment or the manner in which the electronic timepiece 1 is worn.

In addition, the electronic timepiece 1 is provided with the acceleration sensor 18 which detects acceleration by the host device, and the CPU 10, which corresponds to, the controller, performs a skin determination in a case where change in acceleration detected by the acceleration sensor 18 is greater than or equal to the predetermined determination reference. As a result, it is possible to appropriately perform a skin determination on the basis of a quantity of light received that reflects skin lightness, in a state where the electronic timepiece 1 is being worn by a user.

In addition, a method for controlling the electronic timepiece 1 which corresponds to an electronic device according to the present embodiment includes a skin determination step for determining, on the basis of a quantity of light received by the light-receiver 17, whether the skin of the user satisfies a predetermined darkness condition, and a being-worn determination step for determining, on the basis of at least the quantity of light received by the light-receiver 17, whether the user is wearing the host device, the being-worn determination step not being performed in a case of determining in the skin determination step that the darkness condition is satisfied. As a result, it is possible to suitably perform a determination pertaining to a worn state. For example, it is possible to make it less likely for a defect to arise in which a misdetermination that the electronic timepiece 1 is not being worn is made when a user is wearing the electronic timepiece 1.

In addition, the program 111 according to the present embodiment causes the CPU 10 to function as a control means, the CPU 10 corresponding to a computer that is provided in the electronic timepiece 1, which corresponds to an electronic device. The control means performs a skin determination for, on the basis of the quantity of light received by the light-receiver 17, determining whether the user's skin satisfies the predetermined darkness condition. In addition, the control means performs a being-worn determination for determining whether the host device is being worn by the user on the basis of at least a quantity of light received by the light-receiver 17, and does not perform the being-worn determination in a case of determining that a darkness condition is determined in the skin determination. It is possible to suitably perform a determination pertaining to a worn state by using such a program 111 to cause the electronic timepiece 1 to operate. For example, it is possible to make it less likely for a defect to arise in which a misdetermination that the electronic timepiece 1 is not being worn is made when a user is wearing the electronic timepiece 1.

### (Other)

Note that description in the embodiment described above is for an example of an electronic device, a method for controlling an electronic device, and a program that are according to the present invention, and there is no limitation thereto.

For example, the electronic timepiece 1 was exemplified as an electronic device, but there is no limitation to this. For example, the electronic device may be various types of wearable devices such as an activity meter. In addition, it is sufficient if the electronic device is something that has a light-emitter and a light-receiver which are used in a skin determination and a being-worn determination, and is not limited to something capable of pulse detection. In a device that does not perform pulse detection, it is sufficient if a skin determination can be performed by deeming that a user is wearing the electronic device in a case where, for example, change in acceleration is greater than or equal to the above-described determination condition. In addition, a position where the electronic device is worn is not limited to a wrist.

In addition, something that performs a digital display in a dot-matrix format is exemplified as the electronic timepiece 1, but there is no limitation to this, and an analog electronic timepiece that uses a hand to display at least some of information such as the time and a pulse rate may be used.

In addition, description is given by taking an example in which a result of detecting a pulse is used to measure a pulse rate, but there is no limitation to this. A result of detecting a pulse may be used in, inter alia, processing for causing a heartbeat pulse to be displayed or causing a blinking display that correspond to pulsation to be performed, for example.

In addition, description is given by using an example in which a skin determination process is performed and the being-worn determination execution flag 112 is rewritten each time of the pulse rate measurement process, but there is no limitation to this. A result of a skin determination in an initial pulse rate measurement process may be reused in second and subsequent pulse rate measurement processes in, inter alia, a case where it is possible to distinguish the user who is using the electronic timepiece 1 or in a case where usage by a plurality of users is not envisioned. In such cases, it is sufficient if content of the being-worn determination execution flag 112 determined in the skin determination process in the initial pulse rate measurement process are associated with the user and stored in setting data in the memory 11 and, in second and subsequent pulse rate measurement processes, the necessity to execute a being-worn determination is determined on the basis of this setting data.

In the above description, an example of using the non-volatile memory in the memory 11 as a computer-readable medium for a program according to the present invention was disclosed, but there is no limitation to this example. As another computer-readable medium, it is possible to employ an information recording medium such as an HDD, an SSD, a flash memory, or a CD-ROM. In addition, a carrier wave (carrier wave) is applied to the present invention as a medium for providing, via a communication line, data for a program according to the present invention.

In addition, it goes without saying that changes can be made, as appropriate, to the detailed configuration and detailed operation of each component of the electronic timepiece 1 which corresponds to an electronic device according to the embodiment described above, in a scope that does not deviate from the spirit of the present invention.

While some embodiments of the present invention are described, the scope of the present invention is not limited to the embodiments described above, and includes the scope of the invention set forth in the claims and the scope of their equivalents.

The present application is based on Japanese Patent Application No. 2021-051401 filed on March 25, 2021. The specification, claims, and drawings of Japanese Patent Application No. 2021-051401 are entirely incorporated in the present specification by reference.

### INDUSTRIAL APPLICABILITY

By virtue of the present invention, it is possible to suitably perform a determination pertaining to a worn state.

### REFERENCE SIGNS LIST

- 1: Electronic timepiece (electronic device)
- 2: Body
- 3: Belt
- 10: CPU (controller, control means)
- 11: Memory
- 111: Program
- 112: Worn determination execution flag
- 12: Display
- 121: Display screen
- 13: Operation interface
- 131: Operation button
- 141: Oscillator circuit
- 142: Frequency divider
- 143: Timekeeping circuit
- 15: Light-emitter
- 16: Light-emission driver
- 17: Light-receiver
- 18: Acceleration sensor
- 19: A/D converter
- V1: First reference value
- V2: Second reference value

## Claims

1. An electronic device configured to be worn by a user, comprising:
a light-emitter that emits light onto skin of the user;
a light-receiver provided at a position for enabling reception of the light reflected by the skin of the user when the electronic device is being worn by the user and the light-emitter is emitting the light; and
a controller, wherein
the controller:
performs a skin determination for, on the basis of a quantity of light received by the light-receiver, determining whether the skin of the user satisfies a predetermined darkness condition;
performs a being-worn determination for, on the basis of at least the quantity of light received by the light-receiver, determining whether the user is wearing the electronic device; and
does not perform the being-worn determination in response to determining in the skin determination that the darkness condition is satisfied.

2. The electronic device according to claim 1, wherein the controller performs the being-worn determination in response to determining in the skin determination that the darkness condition is not satisfied.

3. The electronic device according to claim 1 or 2, wherein the controller detects a pulse on the basis of change in the quantity of light received by the light-receiver.

4. The electronic device according to claim 3, wherein the controller performs the skin determination on the basis of the quantity of light received by the light-receiver when the pulse is being detected.

5. The electronic device according to any one of claims 1 to 4, wherein the controller determines in the skin determination that the darkness condition is satisfied when the quantity of light received by the light-receiver is less than or equal to a first reference value.

6. The electronic device according to claim 5, wherein
the controller determines in the being-worn determination that the user is wearing the electronic device when the quantity of light received by the light-receiver is greater than a second reference value, and
the first reference value is greater than the second reference value.

7. The electronic device according to any one of claims 1 to 6, further comprising:
an acceleration sensor that detects acceleration of the electronic device,
wherein the controller performs the skin determination in a case where change in acceleration detected by the acceleration sensor is greater than or equal to a predetermined determination reference.

8. A method for controlling an electronic device that is configured to be worn by a user and is provided with a light-emitter that emits light onto skin of the user and a light-receiver provided at a position for enabling reception of the light reflected by the skin of the user when the electronic device is being worn by the user and the light-emitter is emitting the light, the method comprising:
a skin determination step for determining, on the basis of a quantity of light received by the light-receiver, whether the skin of the user satisfies a predetermined darkness condition, and
a being-worn determination step for determining, on the basis of at least the quantity of light received by the light-receiver, whether the user is wearing the electronic device, wherein
the being-worn determination step is not performed in response to determining in the skin determination step that the darkness condition is satisfied.

9. A program executed by a computer provided in an electronic device that is configured to be worn by a user and is provided with a light-emitter that emits light onto skin of the user and a light-receiver provided at a position for enabling reception of the light reflected by the skin of the user when the electronic device is being worn by the user and the light-emitter is emitting the light, the program causing the computer to function as a controller, wherein
the controller:
performs a skin determination for, on the basis of a quantity of light received by the light-receiver, determining whether the skin of the user satisfies a predetermined darkness condition;
performs a being-worn determination for, on the basis of at least the quantity of light received by the light-receiver, determining whether the user is wearing the electronic device; and
does not perform the being-worn determination in response to determining in the skin determination that the darkness condition is satisfied.
